(19)

Europäisches
Patentamt
European
Patent Office
Office européen
des brevets

(11) **EP 2 377 521 A1**

(12) **EUROPEAN PATENT APPLICATION**

(43) Date of publication:
**19.10.2011  Bulletin 2011/42**

(21) Application number: **10157864.9**

(22) Date of filing: **26.03.2010**

(51) Int Cl.:
***A61K 9/20*** *(2006.01)*  ***A61K 31/4184*** *(2006.01)*
***A61K 31/54*** *(2006.01)*

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA ME RS**

(71) Applicant: **Abdi Ibrahim Ilac Sanayi ve Ticaret
Anonim Sirketi
Istanbul (TR)**

(72) Inventors:
• **Farshi, Farhad
  34555 Istanbul (TR)**
• **Avci, Recep
  34555 Istanbul (TR)**
• **Kandemirer, Urun
  34555, Istanbul (TR)**
• **Koc, Fikret
  34555 Istanbul (TR)**
• **Soylemez, Serdar
  34555 Istanbul (TR)**

(54) **Pharmaceutical formulations of telmisartan and diuretic combination**

(57)    This invention is related to tablet in tablet or coated tablet formulations of telmisartan and a diuretic which preferably hydrochlorothiazide.

**EP 2 377 521 A1**

## Description

### Technical Field

This invention is related to tablet in tablet formulations and coated tablet formulations of telmisartan and a diuretic which preferably hydrochlorothiazide.

### Background Art

Telmisartan was firstly disclosed by EP 0552765 B (DR. KARL THOMAE GMBH) 14.08.1996.

EP1467712B (BOEHRINGER INGELHEIM PHARMA GMBH & CO. KG) 12.12.2007 delineates bilayer tablet formulations of telmisartan and hydrochlorothiazide and method of producing said bilayer tablet. Furthermore, the dissolution rate of HCTZ from tablets comprising coated HCTZ in a telmisartan formulation was further reduced due to the get forming properties of the polymer.

PCT/WO2008146178 A (WOCKHARDT RESEARCH CENTRE) 04.12.2008 discloses dosage form includes a layer that includes a tablet of one or more active pharmaceutical ingredients, which is inlayed in the first layer along with other pharmaceutically acceptable excipients, and a second layer that includes one or more active pharmaceutical ingredients optionally with other pharmaceutically acceptable excipients. The distinctive character of WO 20081146178 is that a tablet is inlayed in the first layer. In the second layer, there is no inlayed tablet and second layer alone contains active pharmaceutical ingredient.

PCT/WO 2009154810 A (DR.REDDY'S LABORATORIES LTD. ET.AL) 23.12.2009 discloses a solid pharmaceutical composition comprising at least two active agents, or comprising a single active agent in two or more different forms, comprising a substrate containing a first active agent present in a first tablet, a first tablet being disposed within a second tablet, and a different active agent or a second form of a first active agent deposited onto one or more areas of a surface of a second tablet, present in a layer or between layers of a second tablet, or both. According to WO 2009154810 there are at least three unified tablets and if two different active pharmaceutical ingredients are used, each active pharmaceutical ingredient is posed in its own tablet and if there is a incompatability between tabletted active pharmaceutical ingredients, one tablet is inlayed into the a tablet which includes inert materials.

Both WO 20081146178 and WO 2009154810 disclose the bilayer tablet in which one layer contains inlayed tablet or tablets. However according to our invention, formulation is neither bilayer tablet nor inlayed tablet in which one layer includes inlayed tablet or tablets. As per our invention, formulation is tablet in tablet and there is no inert material layer or active pharmaceutical ingredient layer in which active pharmaceutical ingredient is compatible with active pharmaceutical ingredient of inlayed tablet into the said layer.

### Description of embodiments

In telmisartan formulations, basic agents are used and telmisartan formulation which include basic agent exhibits incompatability with diuretics like HCTZ. Telmisartan needs the incorporation of an alkaline excipient. Diuretics like hydrochlorothiazide, however, degrade in an alkaline environment and reduces dissolution rate of diuretics. According to this invention said incompatability is eliminated by tablet in tablet formulation. This invention does not need inlaying of a tablet of telmisartan which includes basic agent or a tablet of HCTZ into the tablet layer which includes inert materials. Telmisartan tablet which includes basic agent and HCTZ are together disposed as tablet in tablet formulation and said tablet in tablet formulations do not exhibit incompatability.

The composition of the present invention is in the form of a tablet-in-tablet wherein the core tablet includes a diuretic composition and the surrounding outer tablet applied thereover is telmisartan composition or core tablet includes a telmisartan composition and the surrounding outer tablet applied thereover is a diuretic composition. The outer coating layer serves to provide an effective amount of telmisartan or diuretic; while the core tablet provides an effective amount of diuretic or telmisartan.

Tablet in tablet, also known as a "compression coated tablet" comprises an inner tablet that is covered by an outer coat that is compressed onto the inner tablet.

The main advantage of a tablet-in-tablet formulation compared to the bilayer formulation is that outer layer and the inner layer are in a fixed position and cannot be easily separated during storage and handling. Therefore tablet in tablet formulation of telmisartan and a diuretic should be achieved.

In one aspect, this invention embraces unit formula of tablet in tablet and coated tablet which includes telmisartan or pharmaceutically acceptable salts thereof and a pharmaceutically acceptable diuretic. Formulations include a diuretic and telmisartan or pharmaceutically acceptable salts thereof along with pharmaceutical excipients such as disintegrants, diluents, binders, lubricants mixtures thereof and the like.

Diuretics are, but not limited to, chlorothiazide, flumethiazide, benzthiazide, hydrochlorothiazide, hydroflumethiazide, bendroflumethiazide, polythiazide, methycyclothiazide, trichlormethiazide, cyclothiazide and cyclopenthiazide, furosemide, torsemide, bumetanide, piretanide, spironolactone, triamterene, amiloridehiazides, metolazone, dichlorphenamide and the like. Preferably hydrochlorothiazide is used as a duretic.

Disintegrants are, but not limited to, sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose,lower alkylsubstituted hydroxypropyl cellulose, polacrilin potassium, starch, pregelatinized starch, sodium alginate,agar, calcium alginate, tribasic calcium phosphate, dibasic calcium phosphate, corn starch, maize starch, waxy maize starch , potato starch, rice starch, carboxymethyl starches, purified wood cellulose, crosslinked carboxymethyl cellulose, low substituted hydroxypropyl cellulose,hydroxylpropyl starch, guar gum, pectins, cation exchange resins, crosslinked homopolymers of vinylpyrrolidone,magnesium silicates, aluminium silicates and colloidal silicon dioxide, mixtures thereof and the like.

Diluents are, but not limited to, anhydrous lactose,lactose monohydrate, modified lactose, dibasic calcium phosphate, tribasic calcium phosphate, microcrystalline cellulose, silicified microcrystalline cellulose, powdered cellulose, maize starch, pregelatinized starch, calcium carbonate, sucrose, glucose, dextrates, dextrins, dextrose, fructose, lactitol, mannitol, sorbitol, starch, mixtures thereof and the like.

Binders are, but not limited to, polyvinylpyrolidone, copovidone,starches such as pregelatinized starch or plain starch, cellulose derivatives such as hydroxypropylmethylcellulose ,ethylcellulose, hydroxypropylcellulose and carboxymethylcellulose and their salts, gelatine, acacia, agar,alginic acid, carbomer, ceratonia, chitosan, dextrates,dextrin, glycerol dibehenate,guar gum,hypromellose, inulin,magnesium aluminium silicate,maltodextrin,poloxamer,polycarbophil,polydextrose, polyethylene oxide,polymethacrylates,sodium alginate, sucrose, hydrogenated vegetable oil,mixtures thereof and the like.

Lubricants are, but not limited to, magnesium stearate,calcium stearate,hydrogenated castor oil,glyceryl behenate,glyceryl monostearate,glyceryl palmitostearate,leucine,mineral oil, light mineral oil, myristic acid,palmitic acid, polyethylene glycol,potassium benzoate,sodium benzoate,sodium lauryl sulfate,sodium stearyl fumarate,stearic acid, talc, hydrogenated vegetable oil,zinc stearate, magnesium lauryl sulphate,sodium stearyl fumarate, polyethylene glycol, stearic acid, colloidal silicon dioxide , mixtures thereof and the like.

Tablet in tablet formulations can be prepared by dry granulation, wet granulation,direct compression and such conventional technics known by pharmaceutical industry. Preferably the coating layer and the core layer of the tablet-in-tablet dosage form of the invention are formed by wet granulation.

A diuretic and telmisartan can be compressed into tablet-in-tablet through using a press-coater. Tablet in tablet can be produced by using of conventional press-coating technology, such as Kilian™ tablet press.

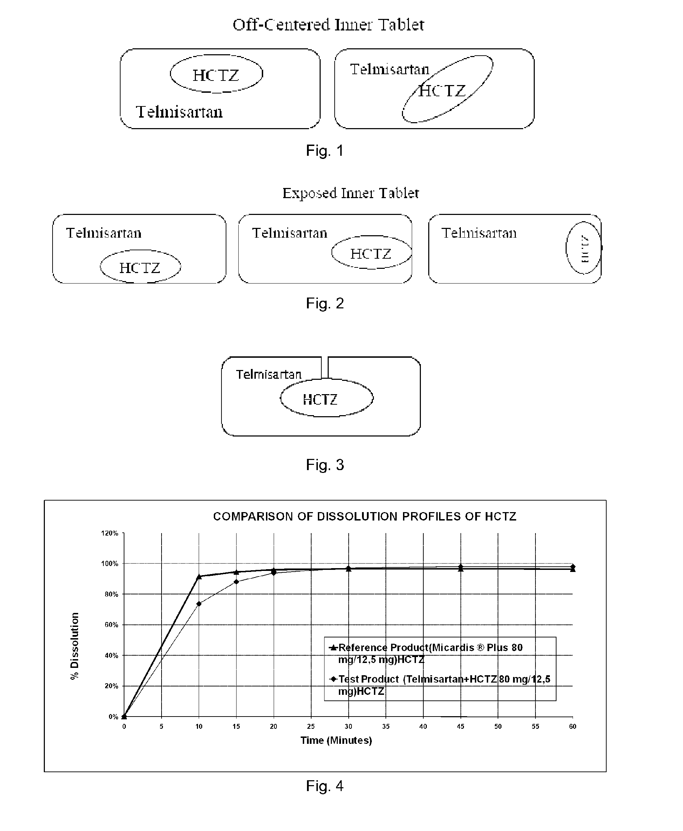

In another aspect, this invention is related to adjusting of dissolution profile of inner tablet through off-centering of inner tablet that is involved by tablet in tablet (Figure 1). Off-centered of the core either vertically or horizontally produces weak edges to reach proper dissolution profiles comply with the dissolution profiles of HCTZ or telmisartan which is included Micardis® Plus tablet. Off-centering can be made by unequal coating, cocking and such technics.

In another aspect of this invention, a part of surface of core tablet is exposed to adjust dissolution profile (Figure 2).

In another aspect this invention is related to adjusting of dissolution profile of inner tablet through piercing or scoring of outer tablet. In this case, outer tablet has one or more passageways or one or more scored regions. (Figure 3). The passageway includes one or more holes or one or more scored regions or one or more holes and one or more scored regions together that penetrate the outer tablet. The passageways are generally formed with a laser. Other conventional technics such as penetration by using of needles can also be used. Passageway provides that the core tablet will begin to release the diuretic agent through the passageway into the intestines

Another aspect of this invention is related to pharmaceutical formulation which includes intragranulation and extragranulation phases for the outer tablet. Accordingly, tablet in tablet formulation comprising following unit formulas:

If inner tablet is diuretic, intragranulation of telmisartan comprising telmisartan or pharmaceutically acceptable salts thereof is from about 5 % to about 30 %, basic agent is from about 2% to about 15%, diluent is from about 30% to about 95% by weight.

Preferably, intragranulation of telmisartan comprising telmisartan or pharmaceutically acceptable salts thereof is 17,04%, basic agent is 6,56 %, diluent is 76, 40% (Table 1).

**Table 1**

| INGREDIENTS | mg | % |
|---|---|---|
| **TELMISARTAN INTRAGRANULATION** | | |
| Telmisa rtan | 80 | 17,04% |
| Basic agent | 30,8 | 6,56% |

(continued)

| INGREDIENTS | mg | % |
|---|---|---|
| TELMISARTAN INTRAGRANULATION | | |
| Diluent | 358,6 | 76,40% |
| Solvent | Evaporated | Evaporated |
| Telmisa rtan Intragra nular Total | 469,4 | 100,00% |

Extragranulation comprising diluent is from about 15 % to about 80 %, binder is from about 5% to about 70%, disintegrant is from about 3% to about 20%.

Preferably, extragranulation comprising diluent is 58,56%, binder is 32,33%, disintegrant is 9,21 % (Table 3).

**Table 3**

| Telmisartan Extragranulation | | |
|---|---|---|
| Ingredients | mg | % |
| Diluent | 63,6 | 58,56% |
| Binder | 35 | 32,23% |
| Disinteg rant | 10 | 9,21% |
| Telmisa rtan Extragr anular Total | 108,6 | 100,00% |

If inner tablet is telmisartan intragranulation of diuretic is from 0,5 % to 10 %, diluent is from 30% to 99% , binder is from 1 % to 10% by weight. Preferably intragranulation of diuretic comprising diuretic is 3,68%, binder is 2,95 %, diluent is 93, 37% (Table 2).

**Table 2**

| INTRAGRANULATION of HCTZ | | |
|---|---|---|
| Ingredients | mg | % |
| HCTZ | 25 | 3,68% |
| Binder | 20 | 2,95% |
| Granulati on solvent | Evaporated | Evaporated |
| Diluent | 633,75 | 93,37% |
| Total | 678,75 | 100,00% |

If inner tablet is telmisartan or its pharmaceutically acceptable salts thereof extragranulation comprising lubricant is from 1 % to 15 %, disintegrant is from 50% to 99 % by weight. Preferably extragranulation comprising lubricant is 4,76%, disintegrant is 95,24% (Table 4).

**Table** 4

| HCTZ EXTRA GRANULATION | | |
|---|---|---|
| Ingredients | mg | % |
| Disintegr ant | 75 | 95,24% |
| Lubricant | 3,75 | 4,76% |
| Total | 78,75 | 100,00% |

In total, outer tablet comprising telmisartan or pharmaceutically acceptable salts thereof is from about 5% to about 25%,basic agent is from about 1 % to about 15 % ,diluent is from about 30% to about 95%,binder is from about 2% to about 20%,disintegrant is from about 0,5 to about 10% by weight of the outer tablet. If there is, coating is not included in calculation. Preferably outer tablet comprising telmisartan or pharmaceutically acceptable salts thereof is 13,84 %,basic agent is 5,33 %, ,diluent is 73,04 %,binder is 6,06%,disintegrant is 1,73 % by weight of the outer tablet. (Table 5).

Table 5

| INGREDIENTS | mg | % |
|---|---|---|
| OUTER TABLET OF TELMISARTAN | | |
| Telmisarta n | 80 | 13,84% |
| Basic agent | 30,8 | 5,33% |
| Diluent | 422,2 | 73,04% |
| Solvent | Evaporated | Evaporated |
| Binder | 35 | 6,06% |
| Disintegra nt | 10 | 1,73% |
| **Outer Tablet of Telmisarta n** | **578** | **100,00%** |

If outer tablet is diuretic, outer tablet comprising diuretic is from 1 % to 15%, diluent is from 30% to 95%, binder is from 1 % to 20%, disintegrant is from 1 % to 20% by weight of the outer tablet. Preferably outer tablet comprising diuretic is 3,32 %, diluent is 84,08 %, binder 2,65%, disintegrant is 9,95% by weight of the outer tablet (Table 6).

Table 6

| OUTER TABLET OF HCTZ | | |
|---|---|---|
| Ingredients | mg | % |
| HCTZ | 25 | 3,32% |
| Binder | 20 | 2,65% |
| Granulation solvent | Evaporated | Evaporated |
| Diluent | 633,75 | 84,08% |
| Disintegrant | 75 | 9,95% |
| **Total** | **753,75** | **100,00%** |

In another aspect, In total, tablet in tablet comprising outer tablet of telmisartan or pharmaceutically acceptable salts thereof is from about 50% to about 95%,inner tablet of diuretic, preferably HCTZ, is from about 5 % to about 50%. Preferably, tablet in tablet comprising outer tablet of telmisartan or pharmaceutically acceptable salts thereof is 85,25%, inner tablet of diuretic, preferably HCTZ, is 14,75% (Table 7).

Table 7

| INGREDIENTS | mg | % |
|---|---|---|
| Telmisartan Outer Tablet | 578 | 85,25 % |
| Hydrochlorothiazide Inner Tablet | 100 | 14,75 % |
| **Tablet in Tablet Total** | **678** | **100,00%** |

If inner tablet is telmisartan, outer tablet of diuretic is from 30% to 95%, inner tablet of telmisartan or pharmaceutically acceptable salts thereof is from 5 % to 50% by weight of the tablet in tablet. Preferably,outer tablet of diuretic is 75,19%, inner tablet of telmisartan or pharmaceutically acceptable salts thereof is 24,81 % (Table 8).

Table 8

| INGREDIENTS | mg | % |
|---|---|---|
| Telmisartan Inner Tablet Core | 250 | 24,81 % |
| Hydrochlorothiazide Outer Tablet | 757,5 | 75,19 % |
| **Tablet in Tablet Total** | **1007,5** | **100,00%** |

Inner tablet comprises , diuretic, preferably HCTZ, is from about 10% to about 40%, binder is from about 0,5% to about 5%,diluents is from about 40% to about 90%,disintegrant is from about 5% to about 25%,lubricant is from about 0,5% to about 7%. Preferably inner tablet comprising diuretic, preferably HCTZ is 25%, binder is 2%,diluent is 61 %, disintegrant is 10%,lubricant is 2% (Table 9).

Table 9

| Tablet Core of HCTZ | | |
|---|---|---|
| **INGREDIENTS** | **mg** | **%** |
| Hydrochlorothiazide | 25 | 25,00% |
| Binder | 2 | 2,00% |
| Granulation Solvent | Evaporated | Evaporated |
| Diluent | 61 | 61,00% |
| Disintegrant | 10 | 10,00% |
| Lubricant | 2 | 2,00% |
| **Total Tablet Weight** | **100** | **100,00%** |

If diuretic is outer tablet, inner tablet comprises telmisartan is from 10% to 70%, binder is from 2 % to 10 %, diluent is from 30% to 80%,disintegrant is from 1% to 15%,lubricant is from 0,1% to 7%, basic agent is from 2% to 30%. Preferably inner tablet comprising telmisartan or pharmaceutically acceptable salts thereof is 32 % , binder is 4,98 % , diluent is 47,20 % ,disintegrant is 3% ,lubricant is 0,50 % , basic agent is 12,32 % (Table 10).

Table 10

| TELMISARTAN TABLET CORE | | |
|---|---|---|
| **Ingredients** | **mg** | **%** |
| Telmisartan | 80 | 32,00% |
| Basic Agent | 30,8 | 12,32% |
| Diluent | 118 | 47,20% |
| Granulation Solvent | Evaporated | Evaporated |
| Binder | 12,45 | 4,98% |
| Disintegrant | 7,5 | 3,00% |
| Lubricant | 1,25 | 0,50% |
| **Total** | **250** | **100,00%** |

If telmisartan is outer tablet, in total tablet in tablet comprising telmisartan or pharmaceutically acceptable salts thereof is from about 5% to about 20%, diuretic preferably HCTZ is from about 1,5% to about 7%, basic agent is from about 1% to about 10%, binder is from about 2% to about 10%,diluents is from about 40% to about 90%,disintegrant is from about 0,5% to about 10%,lubricant is from about 0,01 % to about 5%. Preferably, In total, tablet in tablet comprising telmisartan or pharmaceutically acceptable salts thereof is 11,80% , diuretic preferably HCTZ is 3,69%, basic agent is 4,54%, binder is 5,46%,diluents is 71,27%,disintegrant is 2,95 %,lubricant is 0,29% by weight (Table 11).

**Table 11**

| INGREDIENTS | mg | % |
|---|---|---|
| **Telmisartan Outer Tablet+HCTZ Inner Tablet** | | |
| Telmisartan | 80 | 11,80% |
| Basic agent | 30,8 | 4,54% |
| Diluent | 483,2 | 71,27% |
| Solvent | Evaporated | Evaporated |
| Binder | 37 | 5,46% |
| Lubricant | 2 | 0,29% |
| Disintegrant | 20 | 2,95% |
| Hydrochlorothiazide | 25 | 3,69% |
| **Tablet Total** | 678 | 100,00% |

If telmisartan or pharmaceutically acceptable salts thereof is inner tablet, tablet in tablet totally comprising telmisartan or pharmaceutically acceptable salts thereof is from 3 % to 20%, diuretic is from 0,5% to 10%, basic agent is from 1% to 10%, binder is from 1% to 1 0%,diluent is from 40% to 90%,disintegrant is from 3 % to 20 %,lubricant is from 0,01 % to 5%. Preferably, tablet in tablet totally comprising telmisartan or pharmaceutically acceptable salts thereof is 7,94 %, diuretic is 2,48 %, basic agent is 3,06 %, binder is 3,22 %,diluent is 74,62 %,disintegrant is 8,19 %,lubricant is 0,50% (Table 12).

**Table 12**

| Telmisartan Inner Tablet+HCTZ Outer Tablet | | |
|---|---|---|
| **Ingredients** | **mg** | **%** |
| Hydrochlorothiazide | 25 | 2,48% |
| Binder | 32,45 | 3,22% |
| Granulation solvent | Evaporated | Evaporated |
| Diluent | 751,75 | 74,62% |
| Disintegrant | 82,5 | 8,19% |
| Lubricant | 5 | 0,50% |
| Telmisartan | 80 | 7,94% |
| Basic Agent | 30,8 | 3,06% |
| **Total** | **1007,5** | **100,00%** |

Another aspect of this invention is related to coating of a compressed tablet that includes telmisartan or pharmaceutically acceptable salts thereof with a solution or suspension or dispersion that includes a diuretic or vice versa, namely compressed tablet of diuretic preferably HCTZ may be coated by a solution or suspension or dispersion which involves telmisartan or pharmaceutically acceptable salts thereof. Accordingly: a) One active pharmaceutical ingredient is dissolved alone or with one or more excipients and then other active pharmaceutical ingredient alone or with one or more excipients are coated with said solution. b) One active pharmaceutical ingredient is dispersed alone or with one or more excipients and then other active pharmaceutical ingredient alone or with one or more excipients are coated with said dispersion. c) One active pharmaceutical ingredient is suspended alone or with one or more excipients and then other active pharmaceutical ingredient alone or with one or more excipients are coated with said suspension.

Coated a compressed tablet comprises: - coating layer includes a disintegrant or mixtures of disintegrants in amount of from 5% to 95% by weight of coating layer or - compressed tablet core is exposed or - compressed tablet core is off-centered or - coating layer has one or more passageways which include one or more holes or one or more scored regions or one or more holes and one or more scored regions together that penetrate the coating layer

According to this invention another way of adjusting the dissolution profile other than exposing, off-centering and creating passageway, is to formulate of outer tablet. In this case in outer tablet formulation, a disintegrant or mixtures of disintegrants especially a super disintegrant or mixtures of super disintegrants can be used. Including a disintegrant or mixtures of disintegrants in the outer tablet is vital to adjust of dissolution profile of inner tablet. Otherwise, inner tablet exhibits undesired dissolution profiles since without disintegrant outer tablet will be delayed to be disintegrated, thus inner tablet will be delayed to contact with gastrointestinal liquids. Thus outer tablet is easily disintegrated after ingestion inner tablet easily contact with gastrointestinal tract.

Disintegrants are but not limited to, modified starches,primojel, sodium starch glycolate ,croscarmallose sodium, carboxymethylcellulose calcium and crospovidone, mixtures thereof and the like. Super disintegrants are preferably used.

A disintegrant or mixtures of disintegrants are used in amount of from about 10% to about 95% by weight of the outer tablet.

In yet another aspect of this invention is a tablet of telmisartan or its pharmaceutically acceptable salt thereof and diuretic characterizing in that the pharmaceutical composition has an in vitro dissolution profile with a similarity factor (f2) of at least 50 to 100 compared to the reference (Micardis®Plus tablet, Boehringer Ingelheim) dissolution profile.

The similarity factor f2 is a measurement of the similarity as shown in equation 1.

## (Equation 1- Similarity Factor)

$$f_2 = 50 * \log \frac{100}{\sqrt{1 + \frac{1}{n} \sum_{t=1}^{n} (R_t - T_t)^2}}$$

n: is the number of sampling time points

$R_t$ : is the amount drug released from a *reference* batch at time t

$T_t$: is the amount drug released from a testbatch at time t.

Generally, f2 values greater than 50 ensure sameness of the performance of the reference product and test product.

The pharmaceutical compositions, embedded in this invention, have in vitro dissolution profiles for both HCTZ and telmisartan with a similarity factor (f2) of at least 50 when compared to the dissolution profile of reference (Micardis® Plus tablet) product. Dissolution conditions are phosphate buffer pH 7.5 and 900 ml of a dissolution medium at 37°C $\pm$0.5°C and USP method-II (pedal) and 75 rpm speed.

In another aspect, this invention provides that outer tablet includes intragranulation and extragranulation phases.

In another aspect, this invention embraces a manufacturing method of tablet in tablet formulations which include telmisartan or pharmaceutically acceptable salts thereof and a pharmaceutically acceptable diuretic wherein manufacturing method comprises intragranulation and extragranulation steps.

**Example 1**

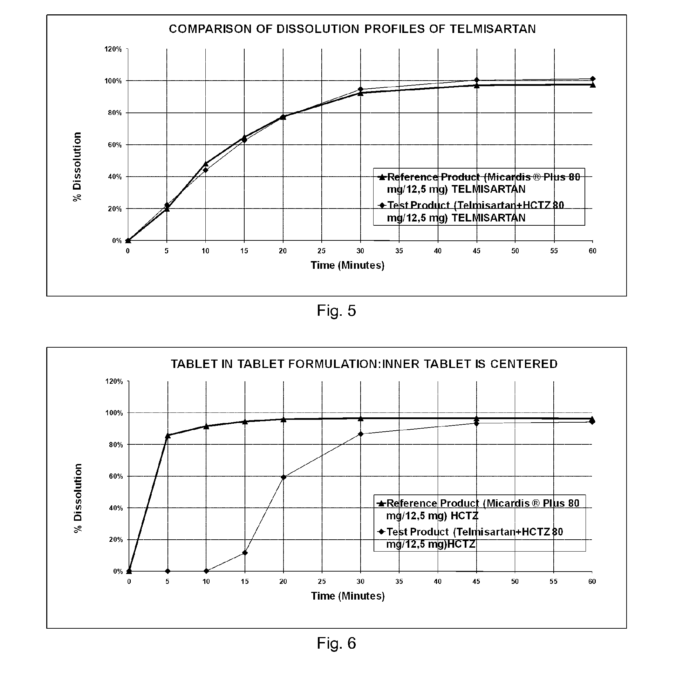

The combination of telmisartan and diuretic preferably HCTZ test tablet, which is produced as defined above, is released in pH 7.5 environment under conditions of 900 ml of a dissolution medium at 37°C $\pm$0.5°C, USP method-II (pedal), 75 rpm basket speed wherein tablet exhibits a dissolution profile (Figure 4, Figure 5 and Table 13). f2 value for telmisartan is 76,3 and for HCTZ is 56,8.

**Table 13**

| Time (Minutes ) | Dissolved % | | | |
|---|---|---|---|---|
| | Micardis® Plus 20 mg Capsule (Reference Product) | | Telmisartan+HCTZ Tablet in Tablet(Test Product) | |
| | HCTZ (12,5 mg) | Telmisartan (80 mg) | HCTZ (12,5 mg) | Telmisartan (80 mg) |
| 10 | 91,6 | 48,2 | 73,8 | 44 |
| 15 | 94,6 | 64,7 | 88,2 | 62,6 |
| 20 | 95,9 | 77,6 | 93,8 | 77,4 |
| 30 | 96,6 | 92,4 | 97,1 | 94,7 |

(continued)

|  | HCTZ (12,5 mg) | Telmisartan (80 mg) | HCTZ (12,5 mg) | Telmisartan (80 mg) |
|---|---|---|---|---|
| 45 | 96,6 | 97,3 | 98 | 100,5 |
| 60 | 96,3 | 97,7 | 98 | 101,2 |

Formulations of this invention provide suitable in vitro dissolution profiles. Comparative examples are given with Figure 4 and Figure 5.

If formulations are not carried out as stipulated above, dissolution profiles of test product and reference product are not eligible. The value of f2 is under 50 of inner tablet. By Figure 6, in a tablet in tablet formulation, centered inner tablet of HCTZ, which does not contain disintegrant as it is defined above,does not exhibit eligible dissolution profile.

**Claims**

1. A pharmaceutical formulation, comprising: (a) an inner tablet core comprising a diuretic and a pharmaceutically acceptable excipient or mixtures of excipients and (b) a compressed coating tablet surrounding said inner tablet core, which comprises an effective amount of telmisartan or a pharmaceutically acceptable salt thereof and one or more pharmaceutically acceptable excipients wherein a part of inner tablet core is exposed or (a) an inner tablet core comprising an effective amount of telmisartan or a pharmaceutically acceptable salt thereof and one or more pharmaceutically acceptable excipients and (b) a compressed coating tablet surrounding said inner tablet core, which comprises a diuretic and a pharmaceutically acceptable excipient or mixtures of excipients wherein a part of inner tablet core is exposed or (a) an inner tablet core comprising a diuretic and a pharmaceutically acceptable excipient or mixtures of excipients and (b) a compressed coating tablet surrounding said inner tablet core, which comprises an effective amount of telmisartan or a pharmaceutically acceptable salt thereof and one or more pharmaceutically acceptable excipients, wherein inner tablet core is off-centered or (a) an inner tablet core comprising an effective amount of telmisartan or a pharmaceutically acceptable salt thereof and one or more pharmaceutically acceptable excipients and (b) a compressed coating tablet surrounding said inner tablet core, which comprises a diuretic and a pharmaceutically acceptable excipient or mixtures of excipients wherein inner tablet core is off-centered posed or (a) an inner tablet core comprising a diuretic and a pharmaceutically acceptable excipient or mixtures of excipients and (b) a compressed coating tablet surrounding said inner tablet core, which comprises an effective amount of telmisartan or a pharmaceutically acceptable salt thereof and one or more pharmaceutically acceptable excipients **characterized in that** outer tablet has one or more passageways which include one or more holes or one or more scored regions or one or more holes and one or more scored regions together that penetrate the outer tablet or (a) an inner tablet core comprising an effective amount of telmisartan or a pharmaceutically acceptable salt thereof and one or more pharmaceutically acceptable excipients and (b) a compressed coating tablet surrounding said inner tablet core, which comprises a diuretic and a pharmaceutically acceptable excipient or mixtures of excipients **characterized in that** outer tablet has one or more passageways which include one or more holes or one or more scored regions or one or more holes and one or more scored regions together that penetrate the outer tablet.

2. According to preceding claim, outer tablet comprising telmisartan or pharmaceutically acceptable salts thereof is from 5% to 25%,basic agent is from 1 to 15,diluent is from 30% to 95%,binder is from 2% to 20%,disintegrant is from 0,5 to 10% by weight of the outer tablet or outer tablet comprising diuretic is from 1% to 15%,diluent is from 30% to 95%,binder is from 1% to 20%,disintegrant is from 1 % to 20% by weight of the outer tablet.

3. According to preceding claim, outer tablet comprising telmisartan or pharmaceutically acceptable salts thereof is 13,84 %,basic agent is 5,33%,binder is 6,06%,disintegrant is 1,73%, diluents is 73,04% by weight of the outer tablet or outer tablet comprising diuretic is 3,32%,diluent is 84,08%,binder is 2,65%,disintegrant is 9,95 % by weight of the outer tablet.

4. According to claim 1, tablet in tablet comprising outer tablet of telmisartan or pharmaceutically acceptable salts thereof is from 50% to 95%,inner tablet of diuretic is from 5 % to 50% by weight of the tablet in tablet or outer tablet of diuretic is from 30% to 95%,inner tablet of telmisartan or pharmaceutically acceptable salts thereof is from 5 % to 50% by weight of the tablet in tablet.

5. According to claim 4, tablet in tablet comprising outer tablet of telmisartan or pharmaceutically acceptable salts thereof is 85,25%, inner tablet of diuretic is 14,75% by weight of the tablet in tablet or outer tablet of diuretic or is 75,19%, inner tablet of telmisartan or pharmaceutically acceptable salts thereof is 24,81 % by weight of the tablet in tablet.

6. According to claim 1 inner tablet comprises diuretic is from 10% to 40%, binder is from 0,5% to 5%,diluents is from 40% to 90%,disintegrant is from 5% to 25%,lubricant is from 0,5% to 7% by weight of the inner tablet or inner tablet comprises telmisartan is from 10% to 70%, binder is from 2 % to 10 %,diluent is from 30% to 80%,disintegrant is from 1% to 15%,lubricant is from 0,1% to 7%, basic agent is from 2% to 30% by weight of the inner tablet

7. According to claim 6, inner tablet comprising diuretic is 25%, binder is 2%,diluent is 61 %,disintegrant is 10%,lubricant is 2% by weight of the inner tablet or inner tablet comprising telmisartan or pharmaceutically acceptable salts thereof is 32 % , binder is 4,98 % ,diluent is 47,20 % ,disintegrant is 3% ,lubricant is 0,50 % , basic agent is 12,32 by weight of the inner tablet.

8. According to claim 1 , if diuretic is inner tablet, tablet in tablet totally comprising telmisartan or pharmaceutically acceptable salts thereof is from 5% to 20%, diuretic is from 1,5% to 7%, basic agent is from 1% to 10%, binder is from 2% to 10%,diluents is from 40% to 90%,disintegrant is from 0,5% to 10%,lubricant is from 0,01 % to 5% by weight of the tablet in tablet or if telmisartan or pharmaceutically acceptable salts thereof is inner tablet, tablet in tablet totally comprising telmisartan or pharmaceutically acceptable salts thereof is from 3 % to 20%, diuretic is from 0,5% to 10%, basic agent is from 1 % to 10%, binder is from 1 % to 1 0%,diluent is from 40% to 90%,disintegrant is from 3 % to 20 %,lubricant is from 0,01 % to 5% by weight of the tablet in tablet

9. According to claim 8 , if diuretic is inner tablet, tablet in tablet totally comprising telmisartan or pharmaceutically acceptable salts thereof is 11,80% , diuretic is 3,69%, basic agent is 4,54%, binder is 5,46%,diluents is 71,27%, disintegrant is 2,95 %,lubricant is 0,29% by weight of the tablet in tablet or if telmisartan or pharmaceutically acceptable salts thereof is inner tablet, tablet in tablet totally comprising telmisartan or pharmaceutically acceptable salts thereof is 7,94 %, diuretic is 2,48 %, basic agent is 3,06 %, binder is 3,22 %,diluent is 74,62 %,disintegrant is 8,19 %,lubricant is 0,50% by weight of the tablet in tablet.

10. A pharmaceutical formulation, comprising: (a) an inner tablet core comprising a diuretic and a pharmaceutically acceptable excipient or mixtures of excipients and (b) a compressed coating tablet surrounding said inner tablet core, which comprises an effective amount of telmisartan or a pharmaceutically acceptable salt thereof and one or more pharmaceutically acceptable excipients **characterized in that** preparing of tablet core has intragranulation and extragranulation steps or (a) an inner tablet core comprising telmisartan or a pharmaceutically acceptable salts thereof and a pharmaceutically acceptable excipient or mixtures of excipients (b) a compressed coating tablet surrounding said inner tablet core, which comprises an effective amount of diuretic and one or more pharmaceutically acceptable excipients **characterized in that** preparing of tablet core has intragranulation and extragranulation steps

11. According to claim 10, intragranulation of telmisartan comprising telmisartan or pharmaceutically acceptable salts thereof is from 5 % to 30 %, basic agent is from 2% to 15%, diluent is from 30% to 95% by weight of the intragranules and extragranulation comprising diluent is from 15 % to 80 %, binder is from 5% to 70%, disintegrant is from 3% to 20% by weight of the extragranules or intragranulation of diuretic comprising diuretic is from 0,5 % to 10 %, diluent is from 30% to 99 % , binder is from 1% to 10% by weight of the intragranules and extragranulation comprising lubricant is from 1 % to 15 %, disintegrant is from 50% to 99 % by weight of the extragranules

12. According to claim 11, intragranulation of telmisartan comprising telmisartan or pharmaceutically acceptable salts thereof is 17,04%, basic agent is 6,56 %, diluent is 76, 40% by weight of the intragranules and extragranulation comprising diluent is 58,56%, binder is 32,33%, disintegrant is 9,21% by weight of the extragranules or intragranulation of diuretic comprising diuretic is 3,68%, binder is 2,95 %, diluent is 93, 37% by weight of the intragranules and extragranulation comprising lubricant is 4,76%, disintegrant is 95,24% by weight of the extragranules

13. A pharmaceutical formulation comprising: a compressed tablet, that includes a diuretic, is coated with a solution or suspension or dispersion of telmisartan or pharmaceutically acceptable salts thereof or a compressed tablet, that includes telmisartan or pharmaceutically acceptable salts thereof, is coated with a solution or suspension or dispersion of a diuretic.

14. According to claim 13 pharmaceutical formulation comprising : - coating layer includes a disintegrant or mixtures of disintegrants in amount of from 5% to 95% by weight of coating layer or - compressed tablet core is exposed or - compressed tablet core is off-centered or - coating layer has one or more passageways which include one or more holes or one or more scored regions or one or more holes and one or more scored regions together that penetrate the coating layer

**15.** According to claim 13 and 14, pharmaceutical formulation is prepared by the following methods: a) telmisartan is dissolved alone or with one or more excipients and then compressed diuretic tablet is coated with said solution or, b) Telmisartan is dispersed alone or with one or more excipients and then compressed diuretic tablet is coated with said dispersion or, c) Telmisartan is suspended alone or with one or more excipients and then compressed diuretic tablet is coated with said suspension or a) diuretic is dissolved alone or with one or more excipients and then compressed telmisartan or pharmaceutically acceptable salts thereof tablet is coated with said solution or, b) diuretic is dispersed alone or with one or more excipients and then compressed telmisartan or pharmaceutically acceptable salts thereof tablet is coated with said dispersion or, c) diuretic is suspended alone or with one or more excipients and then compressed telmisartan or pharmaceutically acceptable salts thereof tablet is coated with said suspension.

**16.** According to preceding claims, the pharmaceutical composition have in vitro dissolution profiles for both HCTZ and telmisartan with a similarity factor (f2) of at least 50 when compared to the dissolution profile of reference (Micardis® Plus tablet) product.

**17.** According to claim 16, dissolution conditions are phosphate buffer pH 7.5 and 900 ml of a dissolution medium at 37°C $\pm$0.5°C and USP method-II (pedal) and 75 rpm speed.

**18.** According to preceding claims diuretic is selected from the group consisting of chlorothiazide, flumethiazide, benzthiazide, hydrochlorothiazide, hydroflumethiazide, bendroflumethiazide, polythiazide, methycyclothiazide, trichlormethiazide, cyclothiazide and cyclopenthiazide, furosemide, torsemide, bumetanide, piretanide, spironolactone, triamterene, amiloridehiazides, metolazone, dichlorphenamide, mixtures thereof.

**19.** According to claim 18 preferred diuretic is hydrochlorothiazide.

**20.** A pharmaceutical formulation, comprising: (a) an inner tablet core comprising a diuretic and a pharmaceutically acceptable excipient or mixtures of excipients and (b) a compressed-outer coating tablet surrounding said inner tablet core, which comprises an effective amount of telmisartan or a pharmaceutically acceptable salt thereof and one or more pharmaceutically acceptable excipients wherein outer tablet includes a disintegrant or mixtures of disintegrants in amount of from 5% to 95% by weight of outer tablet or (a) an inner tablet core comprising an effective amount of telmisartan or a pharmaceutically acceptable salt thereof and one or more pharmaceutically acceptable excipients and (b) a compressed coating tablet surrounding said inner tablet core, which comprises a diuretic and a pharmaceutically acceptable excipient or mixtures of excipients wherein outer tablet includes a disintegrant or mixtures of disintegrants in amount of from10% to 95% by weight of outer tablet

Off-Centered Inner Tablet

Fig. 1

Exposed Inner Tablet

Fig. 2

Fig. 3

Fig. 4

Fig. 5

Fig. 6

Europäisches
Patentamt
European
Patent Office
Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 10 15 7864

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2009/058950 A2 (REDDYS LAB LTD DR [IN]; REDDYS LAB INC DR [US]; PALAPARTHI UMA DEVI [I) 7 May 2009 (2009-05-07) * examples * * claims * ----- | 1-9, 16-19 | INV. A61K9/20 A61K31/4184 A61K31/54 |
| Y,D | WO 2009/154810 A2 (REDDYS LAB LTD DR [IN]; REDDYS LAB INC DR [US]; DHOKE SHRIKANT VITTHAL) 23 December 2009 (2009-12-23) * claims 1,20 * * page 6, line 9 - page 7, line 4 * * figures 1-3 * ----- | 1-9, 16-19 | |
| A | "MICARDIS HCT (TELMISARTAN AND HYDROCHLOROTHIAZIDE) TABLETS, 40MG/12.5 MG 80 MG/12.5 MG AND 80 MG/25 MG", INTERNET CITATION, 19 April 2004 (2004-04-19), XP002365096, Retrieved from the Internet: URL:http://www.fda.gov.medwatch/SAFETY/2004/apr_PI/MicardisHCT_PI.pdf [retrieved on 2006-01-30] * the whole document * ----- | 1-9, 16-19 | |

**TECHNICAL FIELDS SEARCHED (IPC)**

A61K

### INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 11 May 2011 | S. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches
Patentamt

European
Patent Office

Office européen
des brevets

**INCOMPLETE SEARCH
SHEET C**

Application Number

EP 10 15 7864

```
Claim(s) completely searchable:
      1-9

Claim(s) searched incompletely:
      16-19

Claim(s) not searched:
      10-15, 20

Reason for the limitation of the search:

In reply to the invitation to indicate the claims on which the search is
to be based, the applicant failed to supply the requested indication in
due time.
Thus, the search report has been drawn up on the basis of the first
independent claim of each category (Rule 62a(1) EPC): independent product
claim 1 and claims 2-9 (completely), claims 16-19 (partially).
```

**ANNEX TO THE EUROPEAN SEARCH REPORT
ON EUROPEAN PATENT APPLICATION NO.**

EP 10 15 7864

This annex lists the patent family members relating to the patent documents cited in the above-mentioned European search report.
The members are as contained in the European Patent Office EDP file on
The European Patent Office is in no way liable for these particulars which are merely given for the purpose of information.

11-05-2011

| Patent document cited in search report | | Publication date | Patent family member(s) | | Publication date |
|---|---|---|---|---|---|
| WO 2009058950 | A2 | 07-05-2009 | EP<br>US | 2203158 A2<br>2010247649 A1 | 07-07-2010<br>30-09-2010 |
| WO 2009154810 | A2 | 23-12-2009 | NONE | | |

EPO FORM P0459

**EP 2 377 521 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0552765 B
- EP 1467712 B
- WO 2008146178 A
- WO 20081146178 A
- WO 2009154810 A, DR.REDDY